# EUROPEAN PATENT SPECIFICATION

(11) **EP 0 870 033 B1**
(45) Date of publication and mention of the grant of the patent: **21.04.2010**
(21) Application number: 96938869.3
(22) Date of filing: 19.11.1996
(51) Int. Cl.: C12N 15/52, C12N 9/12, A61K 38/48, A61K 31/70, G01N 33/50

(54) **INTEGRIN-LINKED KINASE, ITS INHIBITORS AND METHODS OF MEDICAL TREATMENT USING THESE INHIBITORS, GENE THERAPY AND PSEUDO-SUBSTRATE INHIBITORS**
INTEGRIN-GEKOPPELTE KINASE, IHRE HEMMSTOFFE UND DEREN GEBRAUCH FÜR VERFAHREN ZUR THERAPEUTISCHEN BEHANDLUNG; GENTHERAPIE UND BEREITSTELLUNG VON HEMMSTOFFEN, DIE ALS PSEUDOSUBSTRATE WIRKEN
KINASE LIEE A UNE INTEGRINE, INHIBITEURS DE CETTE KINASE, TRAITEMENT MEDICAL FAISANT APPEL A CES INHIBITEURS, THERAPIE GENIQUE ET INHIBITEURS DU TYPE PSEUDO-SUBSTRATS

(30) Priority: 21.12.1995 US 9074 P
(43) Date of publication of application: 14.10.1998
(73) Proprietor: Sunnybrook & Women's College Health Sciences Centre, North York, Ontario M4N 3MS (CA)
(72) Inventor: Dedhar, Shoukat, Richmond, British Columbia, V7C 5R1 (CA); Hannigan, Greg, Toronto M4C 1W9 (CA)
(74) Representative: Brasnett, Adrian Hugh
(86) International application number: PCT/CA1996/000760
(87) International publication number: WO 1997/023625

(56) References cited:
- EP-A- 0 616 032
- WO-A-94/21781
- WO-A-96/36642
- 86TH ANNUAL MEETING OF THE AMERICAN ASSOCIATION FOR CANCER RESEARCH, March 1995, TORONTO, ONTARIO, CANADA, page 361 XP000673939 HANNIGAN, G.E., ET AL.: "CLONING OF A NOVEL PROTEIN KINASE ASSOCIATED WITH BETA1-INTEGRIN CYTOPLASMIC TAILS"
- MOLECULAR BIOLOGY OF THE CELL, vol. 6, no. SUPPL., November 1995, page 2244 XP000673935 HANNIGAN,G.E., ET AL.: "OVEREXPRESSION OF A NOVEL INTEGRIN LINKED KINASE (ILK) INDUCES A TRANSFORMED PHENOTYPE AND CYCLIN D1 EXPRESSION"
- THE JOURNAL OF BIOLOGICAL CHEMISTRY, vol. 270, no. 1, 6 January 1995, pages 269-273, XP002031634 MORINO, N., ETAL . : "MATRIX/INTEGRIN INTERACTION ACTIVATES THE MITOGEN-ACTIVATED PROTEIN KINASE, p44erk-1 AND p42erk-2"
- EMBL SEQUENCE DATA LIBRARY, 25 October 1995, HEIDELBERG, GERMANY, XP002031635 HILLIER, L. , ET AL.: "THE WASHU-MERCK EST PROJECT"
- CURRENT OPINION IN CELL BIOLOGY, vol. 8, no. 5, October 1996, pages 657-669, XP000673950 DEDHAR,S. AND HANNIGAN,G.E.: "INTEGRIN CYTOPLASMIC INTERACTIONS AND BIDIRECTIONAL TRANSMEMBRANE SIGNALLING"
- NATURE, vol. 379, 4 January 1996, pages 91-96, XP002031636 HANNIGAN, G.E., ET AL . : "REGULATION OF CELL ADHESION AND ANCHORAGE-DEPENDENT GROWTH BY A NEW BETA1-INTEGRIN-LINKED PROTEIN KINASE"
- WU CHUANYUE; KEIGHTLEY SARAH Y; LEUNG-HAGESTEIJN CHUNGYEE; RADEVA GALENA; COPPOLINO MARC; GOICOECHEA SILVIA; MCDONALD JOHN A;: "Integrin-linked protein kinase regulates fibronectin matrix assembly, E-cadherin expression, and tumorigenicity" JOURNAL OF BIOLOGICAL CHEMISTRY, vol. 273, no. 1, 2 January 1998 (1998-01-02), pages 528-536,
- NOVAK ANTON; HSU SHU-CHI; LEUNG-HAGESTEIJN CHUNGYEE; RADEVA GALINA PAPKOFF JACKIE; MONTESANO ROBERTO; ROSKELLEY CALVIN; GROSSCHEDL: "Cell adhesion and the integrin-linked kinase regulate the LEF-1 and beta-catenin signaling pathways" PROCEEDINGS OF THE NATIONAL ACADEMY OF SCIENCES OF THE UNITED STATES OF AMERICA, vol. 95, no. 8, 14 April 1998 (1998-04-14), pages 4374-4379,
- PERSAD SUJATA; ATTWELL SARAH; GRAY VIRGINIA; DELCOMMENNE MARC; TROUSSARD ARMELLE; SANGHERA JASBINDER; DEDHAR SHOUKAT: "Inhibition of integrin-linked kinase (ILK) suppresses activation of protein kinase B/Akt and induces cell cycle arrest and apoptosis of PTEN-mutant prostate cancer cells" PROCEEDINGS OF THE NATIONAL ACADEMY OF SCIENCES OF THE UNITED STATES OF AMERICA, vol. 97, no. 7, 28 March 2000 (2000-03-28), pages 3207-3212,
- TAN CLARA; COSTELLO PENNY; SANGHERA JASBINDER; DOMINGUEZ DAVID; BAULIDA JOSEP; GARCIA DE HERREROS ANTONIO; DEDHAR SHOUKAT: "Inhibition of integrin linked kinase (ILK) suppresses beta-catenin-Lef/Tcf-dependent transcription and expression of the E-cadherin repressor, snail, in APC-/- human colon carcinoma cells" vol. 20, no. 1, 4 January 2001 (2001-01-04), pages 133-140,
- YOUNES MAHER N; KIM SEUNGWON; YIGITBASI ORHAN G; MANDAL MAHITOSH; JASSER SAMAR A; YAZICI YASEMIN DAKAK; SCHIFF BRADLEY A; ET AL.: "Integrin-linked kinase is a potential therapeutic target for anaplastic thyroid cancer" MOLECULAR CANCER THERAPEUTICS, vol. 4, no. 8, May 2005 (2005-05), pages 1146-1156,
- TAN C; CRUET-HENNEQUART S; TROUSSARD A; FAZLI L; COSTELLO P; SUTTON K; WHEELER J; GLEAVE M; SANGHERA J; DEDHAR S: "Regulation of tumor angiogenesis by integrin-linked kinase (ILK)" CANCER CELL, vol. 5, no. 1, January 2004 (2004-01), pages 79-90,
- TAN CLARA; MUI ALICE; DEDHAR SHOUKAT: "Integrin-linked kinase regulates inducible nitric oxide synthase and cyclooxygenase-2 expression in an NF-kappaB-dependent manner" JOURNAL OF BIOLOGICAL CHEMISTRY, vol. 277, no. 5, 1 February 2002 (2002-02-01), pages 3109-3116,
- LIU EMERSON; SINHA SUMITA; WILLIAMS CHRISTINE; CYRILLE MARCOLI; HELLER ERIC; SNAPPER SCOTT B; GEORGOPOULOS KATIA; ST-ARNAUD RENE;: "Force Thomas; Dedhar Shoukat; Gerszten Robert ETargeted deletion of integrin-linked kinase reveals a role in T-cell chemotaxis and survival" MOLECULAR AND CELLULAR BIOLOGY, vol. 25, no. 24, December 2005 (2005-12), pages 11145-11155,
- HANNIGAN GREGORY; TROUSSARD ARMELLE A; DEDHAR SHOUKAT: "Integrin-linked kinase: A cancer therapeutic target unique among its ILK" NATURE REVIEWS CANCER, vol. 5, January 2005 (2005-01), pages 51-63,
- CHEN YOUHAI; INOBE JUN-ICHI; WEINER HOWARD L: "Induction of oral tolerance to myelin basic protein in CD8-depleted mice: Both CD4+ and CD8+ cells mediate active suppression" JOURNAL OF IMMUNOLOGY, vol. 155, no. 2, 1995, pages 910-916,
- LIAO LAN; JAKEN SUSAN: "Effect of alpha-protein kinase C neutralizing antibodies and the pseudosubstrate peptide on phosphorylation, migration, and growth of REF52 cells" CELL GROWTH AND DIFFERENTIATION, vol. 4, no. 4, April 1993 (1993-04), pages 309-316,

## Description

### Background

Proteins of the extracellular matrix (ECM) act to influence fundamental cell and tissue behaviours. ECM regulates cell structure, growth, survival, differentiation, motility and, at the organismal level, proper development. ECM proteins interact with cells via a class of cell membrane-spanning receptors called integrins. ECM is a biological signal, and the integrin receptor is a specific transducer (i.e. across the cell's plasma membrane) of this signal. Integrins are also important in proliferative disorders, mediating such processes as wound healing and inflammation, angiogenesis, as well as tumour migration and invasion.

A major biochemical response to ECM-integrin interactions is elevation of an enzymatic activity known as protein phosphorylation. Phosphorylation is important in signal transduction mediated by receptors for extracellular biological signals such as growth factors or hormones. For example, many cancer causing genes (oncogenes) are protein kinases, enzymes which catalyze protein phosphorylation reactions, or are specifically regulated by phosphorylation. In addition, a kinase can have its activity regulated by one or more distinct protein kinases, resulting in specific signaling cascades.

Research on signal transduction over the years has clearly established the importance of direct, protein-protein interactions in the cytoplasm as a major mechanism underlying the specification of, signaling pathways. These interactions can, in part, be those between a receptor and a cytoplasmic protein kinase, or between a protein kinase and its substrate molecule(s).

A number of known protein kinases, such as mitogen-activated kinase (MAPK), focal adhesion kinase (FAK), and protein kinase C (PKC) have their kinase activity stimulated by integrin-ECM interaction, although no cellular protein kinase has been identified to date, which has been demonstrated to bind to an integrin molecule under physiological conditions. As such is the case, the direct molecular connection between integrins and the ECM-induced phosphorylation of cellular proteins is unclear.

As such is the case, if the direct molecular connection between integrins and the ECM-induced phosphorylation of cellular proteins were determined, products which modulated that connection would be useful therapeutics. These products could be used to modulate cell growth, cell adhesion, cell migration and cell invasion. If it were determined that a specific kinase regulates integrin function, products that regulate (for example, inhibit) the activity of that kinase could be used for the treatment of cancer, leukemia, solid tumors, chronic inflammatory disease, arthritis and osteoporosis, among other indications.

In 86th Annual Meeting of the American Association for Cancer Research March 2005, page 361, XP000673939, Hannigan et al, and in Molecular Biology of the Cell 6, Nov 2005, page 2244, XP000673935 Hannigan et al, the cloning of a protein kinase is described. In the former a cDNA is described which possesses homology to the serine/threonine and tyrosine kinase catalytic domain of protein kinases and which recognises a 2kb mRNA in cell lines and human tissues.

### Summary of the Invention

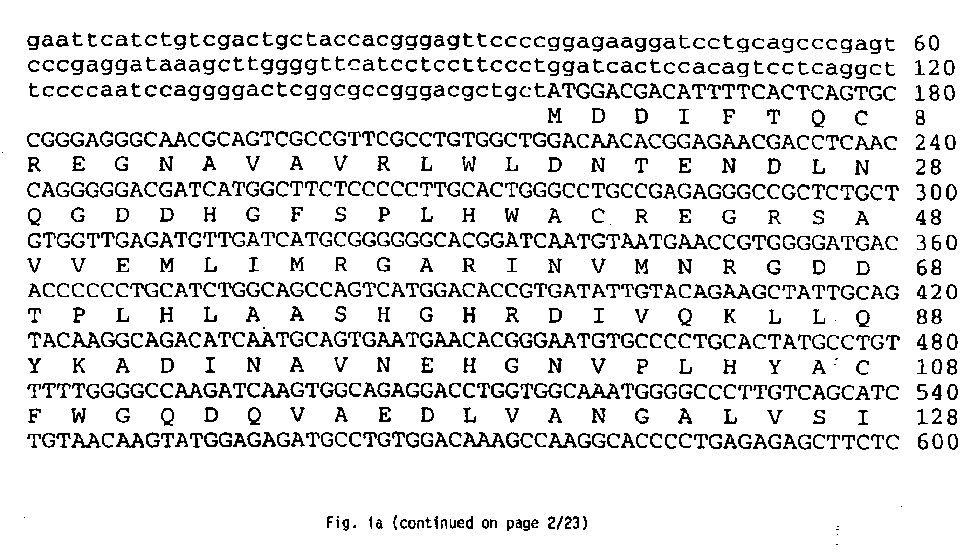

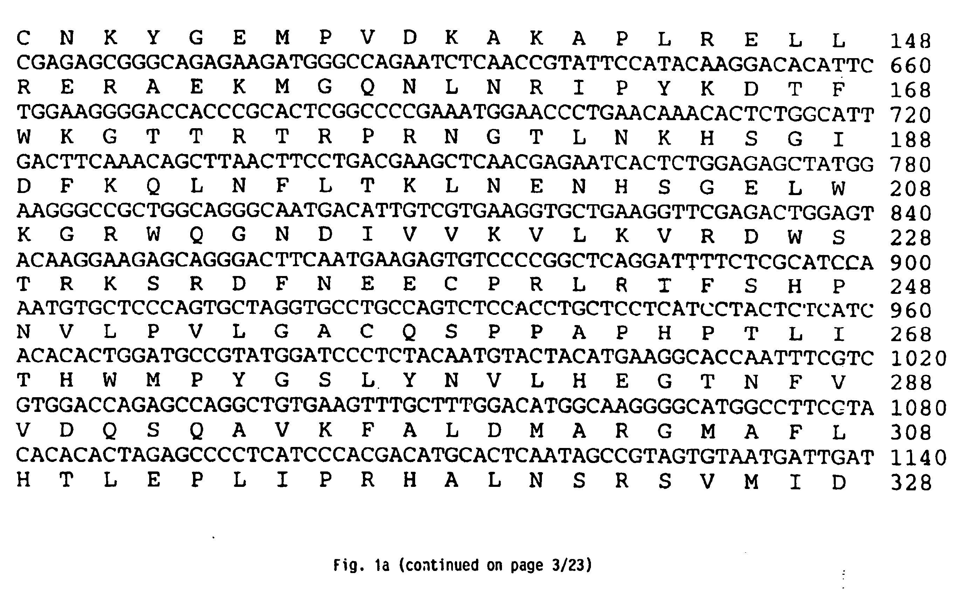

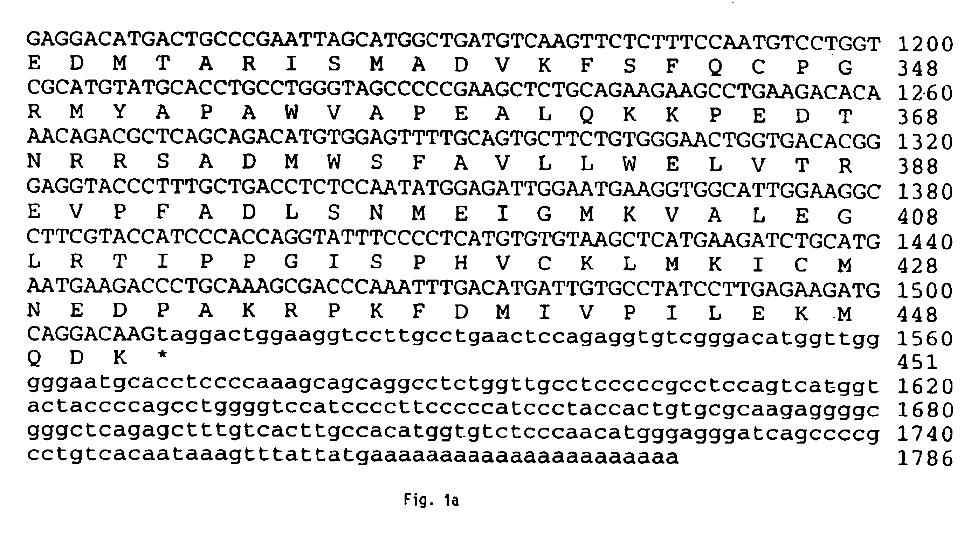

The invention relates to an inhibitor of integrin linked kinase selected from:
(a) an antibody to the ILK molecule encoded by the nucleic acid sequence of Figure 1A which is capable of inhibiting ILK function; and
(b) a synthetic anti-sense DNA oligonucleotide that is complementary to Figure 1 A and capable of inhibiting ILK function.

An isolated and purified serine/threonine kinase which is an integrin-linked kinase, designated "ILK" is described in this application. ILK binds to the cytoplasmic portion of the β₁ integrin molecule in a living cell, providing the first physiological evidence for interaction between an integrin and a protein kinase.

ILK can be used to modulate cell growth, modulate cell adhesion, modulate cell migration and modulate cell invasion. An amino acid sequence of ILK and an isolated nucleotide molecule encoding the amino acid sequence are part of this invention. The molecule could be cDNA, sense DNA, anti-sense DNA, single DNA, double stranded DNA. mRNA of integrin-linked kinase is part of this invention. The molecule could be a nucleotide molecule encoding an impaired amino acid sequence of a serine/threonine kinase.

Inhibitors of ILK activity as claimed are part of this invention. Inhibitors include (1) screens aimed at DNA, RNA or ILK structural components e.g. antisense ILK (i.e. synthetic DNA oligonucleotide comprising the complementary nucleotide sequence of the ILK coding region, designed to specifically target the ILK mRNA complement), (2) pseudo-substrate inhibitors, such as a peptide which mimics a substrate sequence for ILK, and (3) drugs which specifically inhibit ILK activity. These drugs may be directed at either the kinase or ankyrin repeat domains. An inhibitor may include an antibiotic, a natural or mimetic substrate for the integrin-linked kinase, and a first nucleotide molecule which binds to a second nucleotide molecule of the kinase. The second nucleotide molecule may be mRNA, cDNA, sense DNA, antisense DNA, single-stranded and double-stranded DNA.

The invention includes an inhibitor of integrin linked kinase as herein defined for use in a method of treating a disease in a mammal selected from cancer and chronic inflammatory diseases.

The method may include gene therapy, for example, the delivery of a gene or cDNA by any vector (viral or non-viral) .

The carrier for the inhibitor, substrate or molecule would be a pharmaceutically acceptable carrier, diluent or excipient. In the case of a nucleotide molecule, a carrier could be liposomes.

Diagnostics of ILK activity are part of this invention. Diagnostics include nucleotide molecules of ILK, ILK or its inhibitors. DNA-based reagents derived from the nucleotide sequence of ILK and antibodies against ILK screen biopsy-derived samples of amplified ILK DNA, or increased expression of ILK mRNA or protein.

Assays which screen drugs which specifically inhibit ILK activity are included within this invention. These assays may be based on the DNA, mRNA or amino acid sequences of ILK.

The invention includes a pharmaceutical comprising an inhibitor of ILK activity together with a carrier, for modulating cellular activity.

### Figures

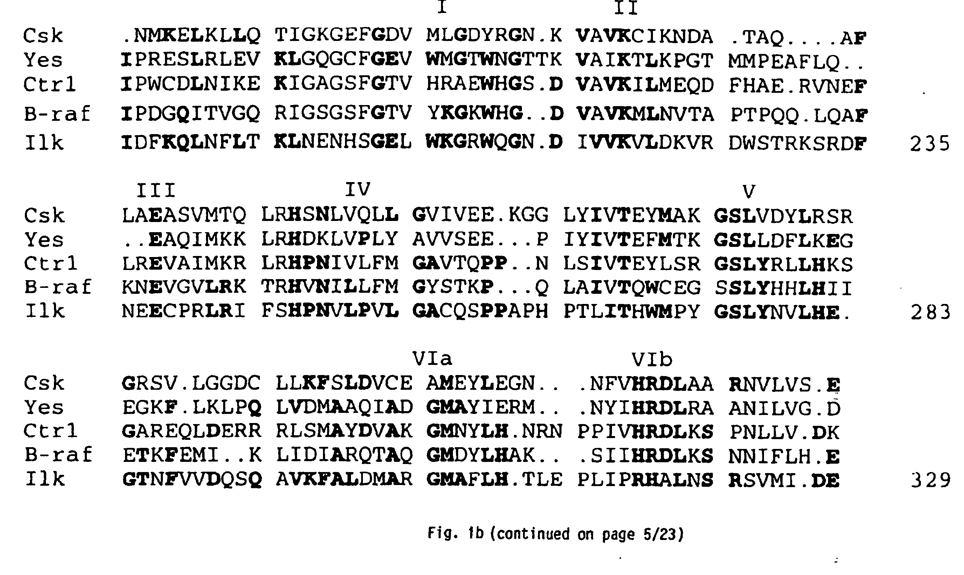

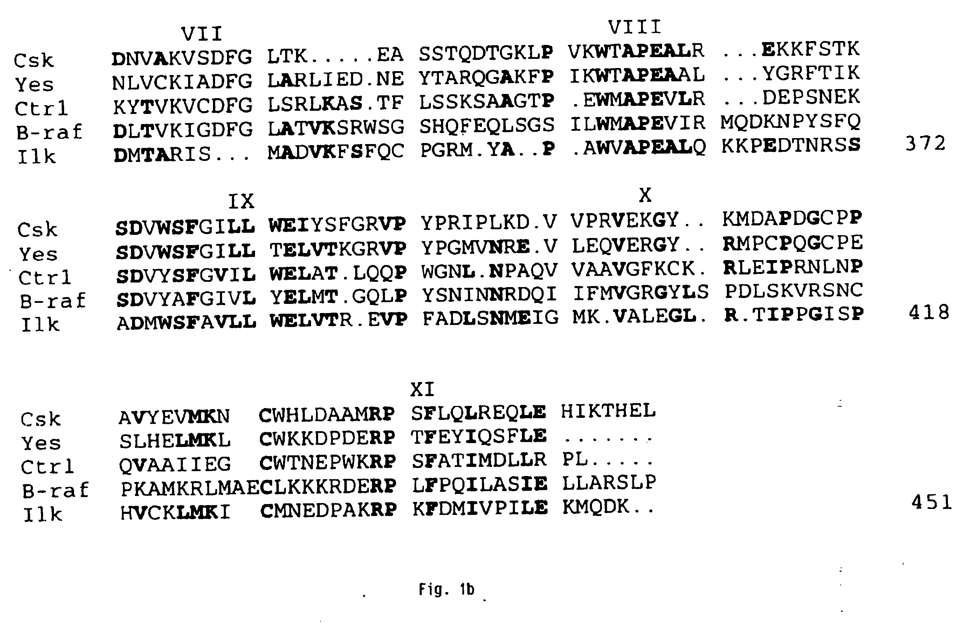

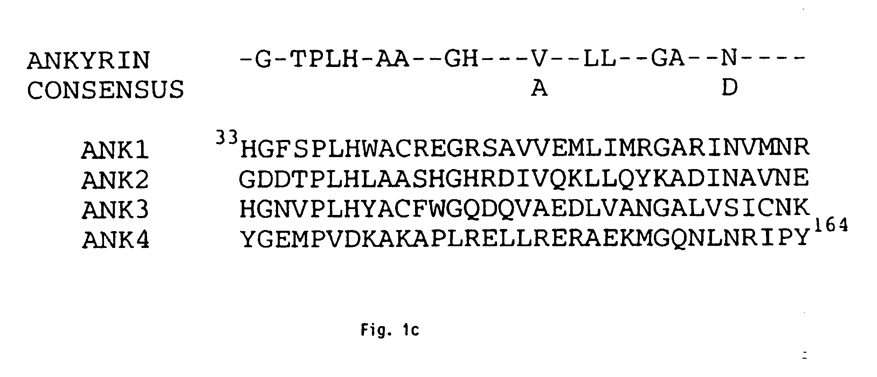

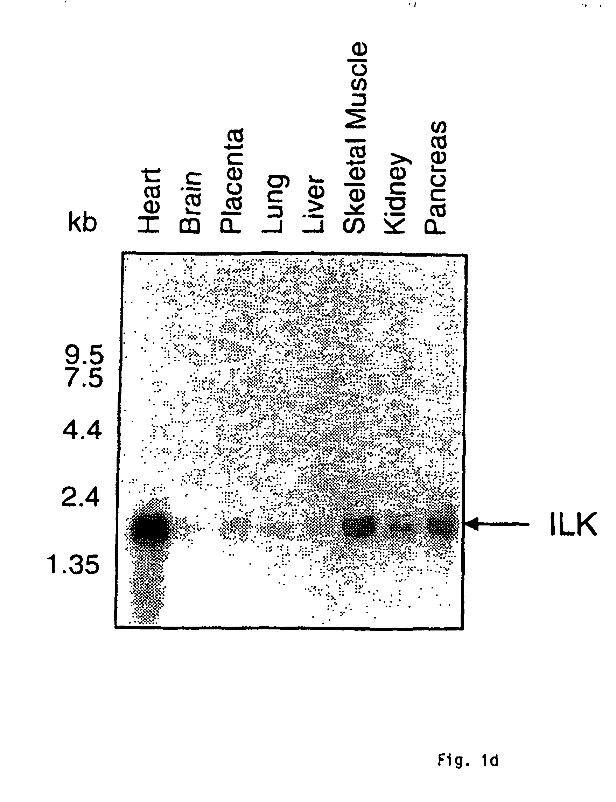

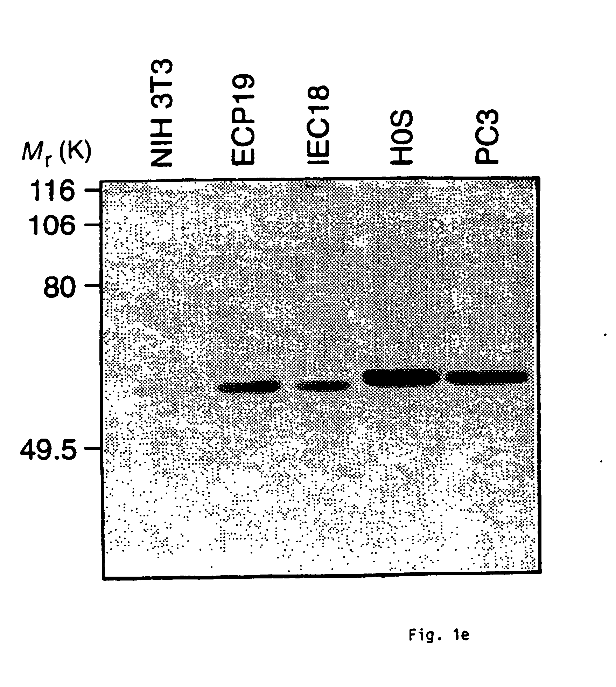

**FIG. 1** Yeast two-hybrid cloning, characterization, and expression of ILK. **a,** The full length ILK cDNA. **b,** Homology with protein kinase subdomains I to XI. **c,** Amino acid residues comprising ankyrin repeats. **d,** BIT-9 used to probe RNA from human tissues. **e,** Analysis of whole cell lysates of mouse, rat and human cell lines.

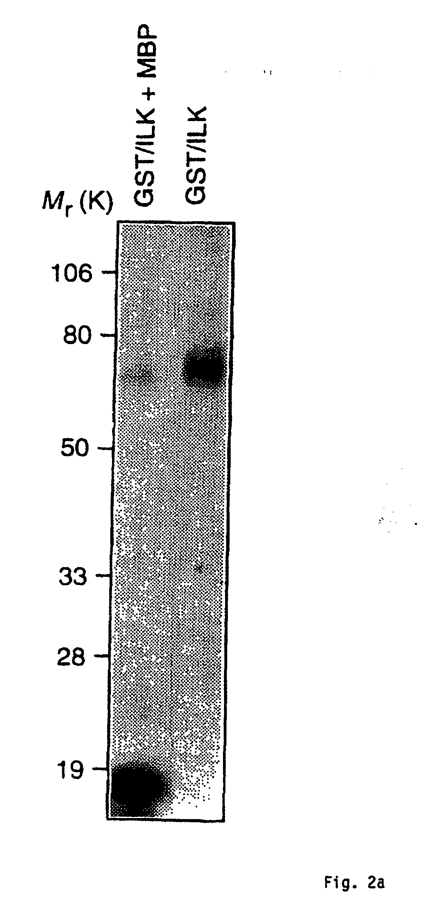

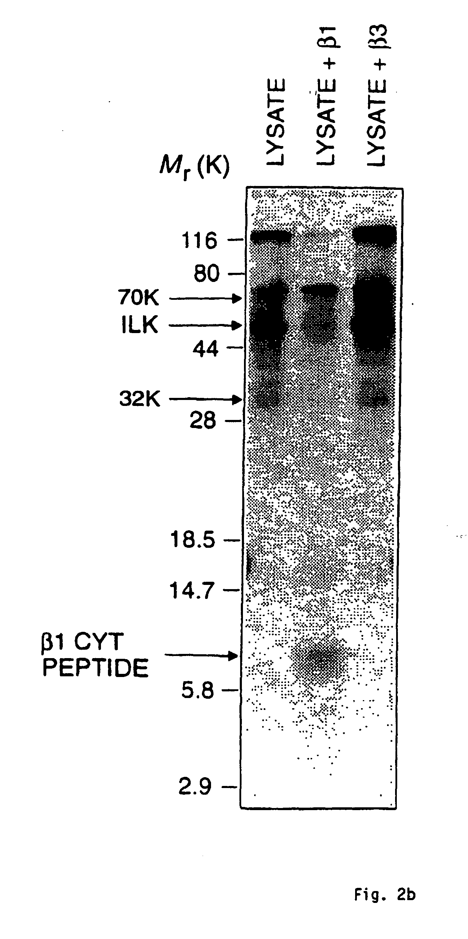

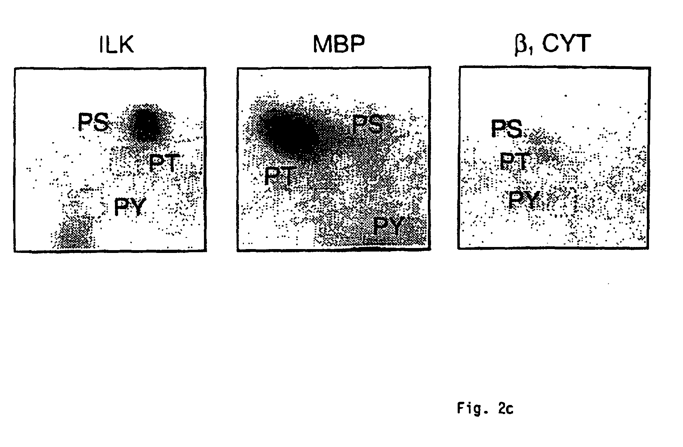

**FIG. 2** *In vitro* and immune-complex kinase assays. **a,** In vitro kinase reactions. **b,** Immune complexes. **c,** ³²P-labelled products isolated and analyzed for phosphoamino acid content.

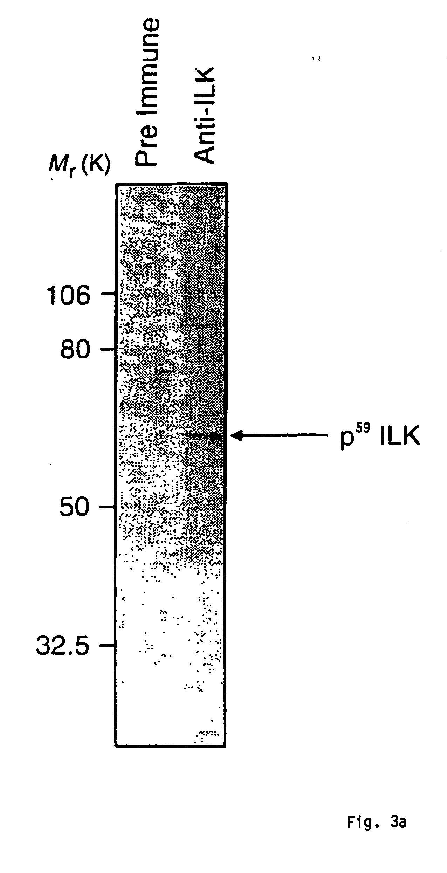

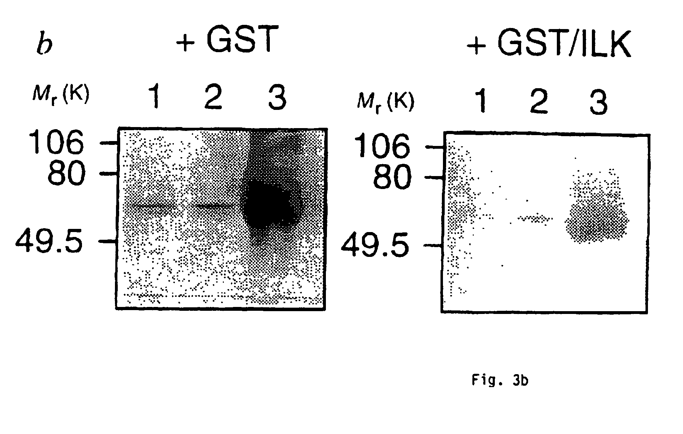

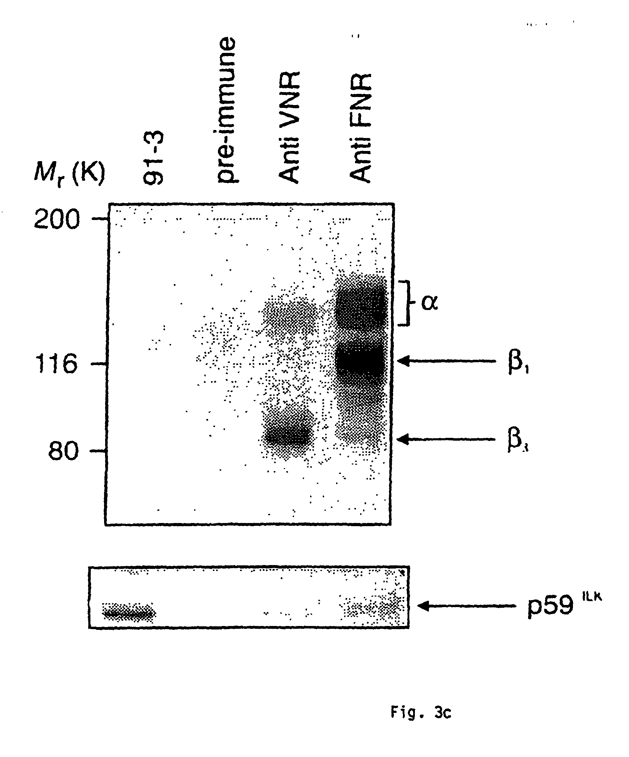

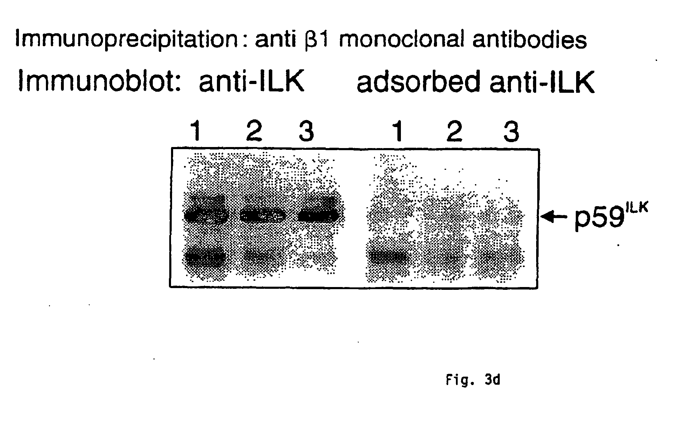

**FIG. 3** Antibodies to GST-ILR¹³² recognize p59^{ILK} in integrin co-immunoprecipitations. **a,** Unfractionated polyclonal anti-ILK sera specifically recognize a ³⁵S-methionine, metabolically-labelled cellular protein. **b,** Affinity-purified antibody was adsorbed with GST-ILK agarose-GST. **c,** Polyclonal anti-integrin antibodies used to precipitate surface-biotinylated integrins from PC3 cells,. **d,** Anti-β₁ monoclonal antibodies were used in co-precipitation analyses of lysates of PC3.

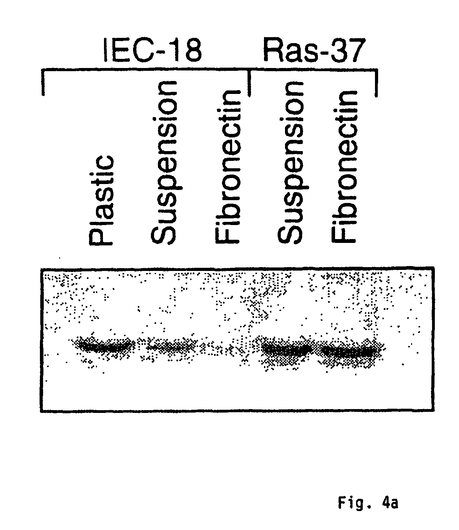

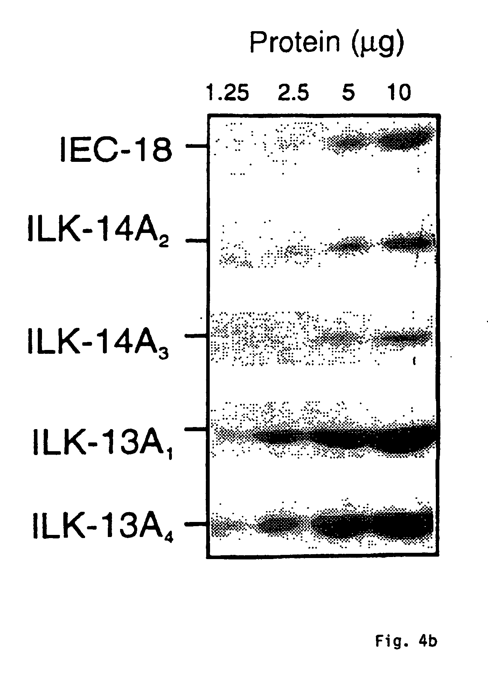

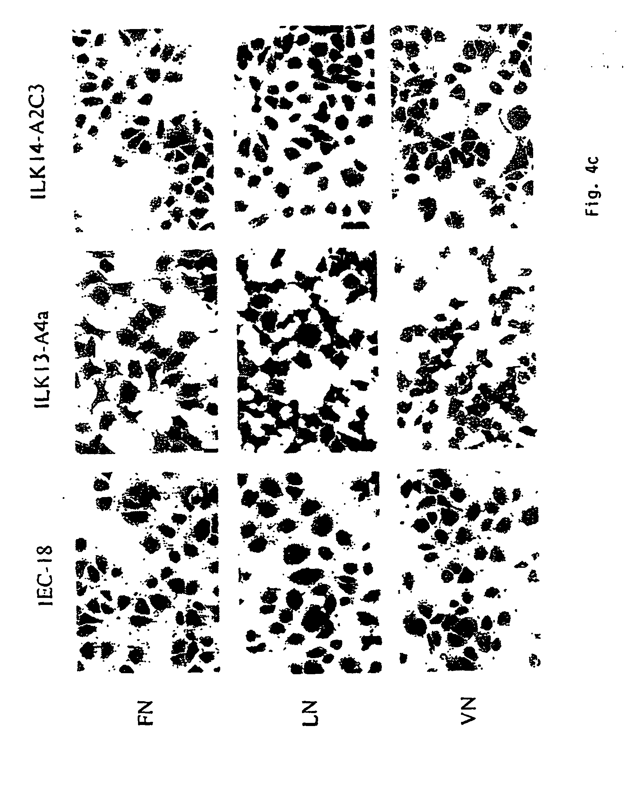

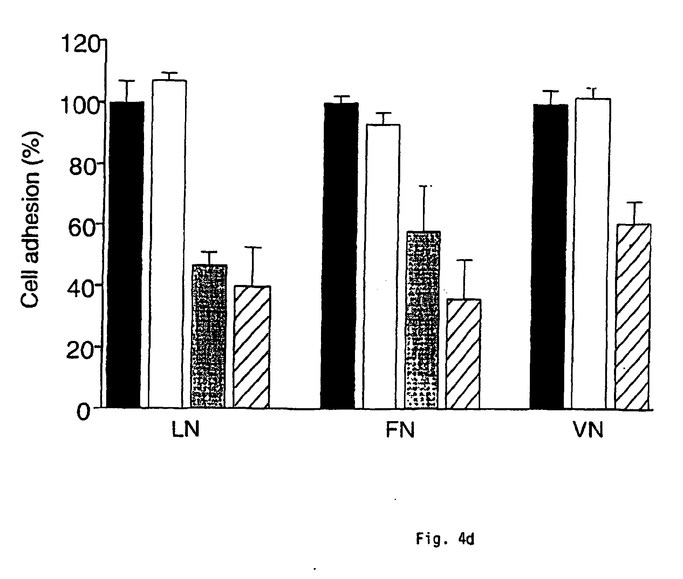

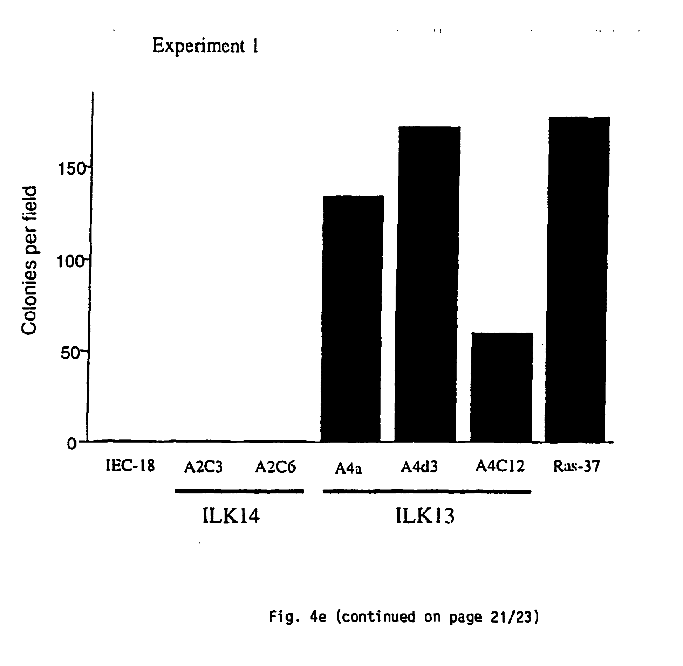

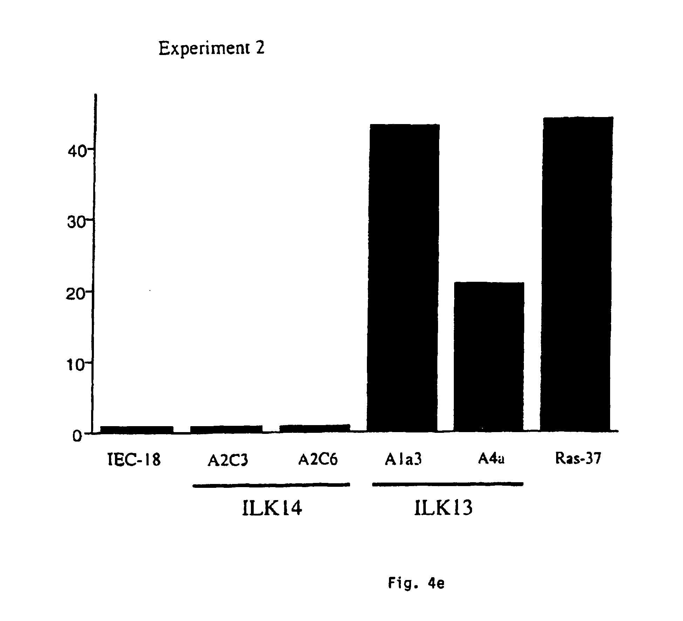

**FIG. 4** Modulation of ILK kinase activity by ECM components. **a,** ILK phosphorylation of MBP was assayed. **b,** Expression levels of p59^{ILK}. **c,** Representative p59^{ILK} overexpressing clone ILK13-A4a on the ECM substrates. **d,** Adhesion of the ILK overexpressing clones to LN, FN and VN was quantified. **e,** ILK13, p59^{ILK} overexpressing clones were assayed for colony growth.

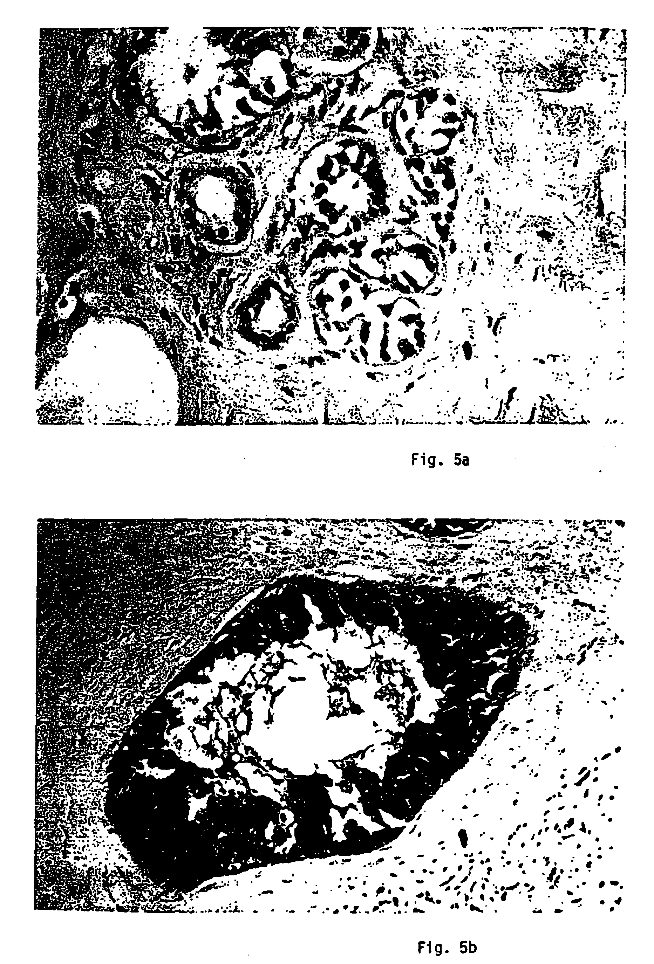

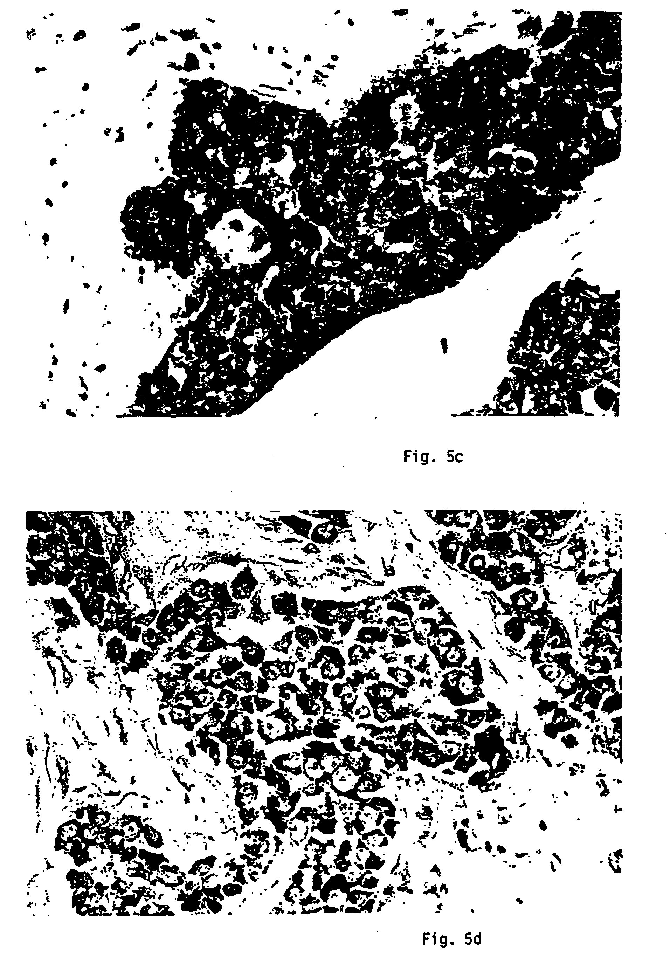

**FIG. 5** Expression of ILK in human breast carcinomas. **a,** Normal region of breast tissue. b, Ductal carcinoma *in situ*. **c,d,** Invasive carcinoma.

### Detailed Description of Preferred Embodiments

By this invention, we have shown the physical linkage between integrin and ILK. More importantly, we have shown that dysregulated expression of ILK protein modulates the function of integrins, thus providing a biological link between ILK and integrin. Dysregulated expression of ILK modulates cell growth, cell adhesion, cell migration and cell invasion. Hence, products that inhibit the activity of dysregulated expression of ILK have a therapeutic effect in the treatment of cancer, leukemia, solid tumors, chronic inflammatory disease, arthritis and osteoporosis, among other indications.

The ILK protein is encoded by a 1.8 kilobase pair messenger RNA (1.8 kb mRNA). The sequence of this mRNA was used to deduce the primary amino acid sequence of the protein, which has a predicted molecular weight of 50 kiloDaltons (kDa). The recombinant protein migrates on analytical polyacrylamide electrophoresis gels with an apparent molecular weight of 59 kDa, in rough agreement with the predicted size. The deduced structure of the ILK protein (hereinafter p59ILK) revealed two functional domains, identified by comparison of the ILK sequence against those found in current protein databases. These are the catalytic domain, responsible for phosphotransferase activity (kinase domain), and a nonoverlapping domain in the amino terminus, comprised of four contiguous ankyrin-like repeats.

The function of ankyrin repeats in ILK is to mediate protein-protein interactions. The ILK ankyrin repeat domain is not required for the binding of p59ILK to integrin, and it presumably mediates the interaction of p59ILK with another cellular protein(s). Thus, p59ILK bridges integrin(s) in the plasma membrane, with intracellular proteins active in regulating the cell's response to ECM signals. These proteins are likely to be located in the cytoplasm, or as part of the cell's structural framework (cytoskeleton), but are as yet unidentified.

The novelty of ILK lies in key structural and functional features of the enzyme. Structurally, it represents an unusual molecular architecture, in that a protein kinase and an ankyrin repeat domain are contained within the same protein. The kinase domain is very conserved (i.e. similar) to other kinase sequences in existing databases, and can be divided into typical subdomains (I through XI), based on this conserved structure. However one amino acid in particular is not present in ILK, which is present in a specific context, in subdomain VIb of all other protein kinase domains. Despite this unique structural feature, ILK clearly acts as a protein kinase, and thus could represent a prototype member of a new subfamily of protein kinase molecules.

The commercial potential of ILK is directly linked to its regulation of integrin extracellular activity (ECM interactions) from inside the cell, via its direct interaction with the integrin subunit (known in the integrin field as "inside-out" signalling). Interfering with ILK activity allows the specific targeting of integrin function, while leaving other essential signalling pathways intact. Moreover, increasing the levels of cellular ILK activity short circuits the normal requirement for adhesion to ECM (i.e. integrin function) in regulating cell growth. Thus, inhibiting ILK activity would inhibit anchorage-independent (i.e. cancerous) cell growth.

Thus, from a therapeutics point of view, inhibiting ILK activity has a therapeutic effect on a number of proliferative disorders, including inflammation and cancer. Inhibition is achieved in a number of ways: (1) with screens aimed at DNA, RNA of ILK or ILK structural components e.g. antisense ILK (i.e. synthetic DNA oligonucleotide comprising the complementary nucleotide sequence of the ILK coding region, designed to specifically target the ILK mRNA complement), (2) pseudo-substrate inhibitors, for example, a peptide which mimics a substrate for ILK, or (3) by assaying inhibition of ILK activity in an ILK-based functional assay e.g. in vitro or in vivo ILK kinase activity.

Knowledge of the 3-dimensional structure of ILK, derived from crystallization of purified recombinant ILK protein, leads to the rational design of small drugs which specifically inhibit ILK activity. These drugs may be directed at either the kinase or ankyrin repeat domains.

From a diagnostics perspective, DNA-based reagents derived from the sequence of ILK, e.g. PCR primers, oligonucleotide or cDNA probes, as well as antibodies against p59ILK, are used to screen biopsy-derived tumours or inflammatory samples e.g. arthritic synovium, for amplified ILK DNA, or increased expression of ILK mRNA or protein. DNA-based reagents are designed for evaluation of chromosomal loci implicated in certain diseases e.g. for use in loss-of-heterozygosity (LOH) studies, or design of primers based on ILK coding sequence.

Having mapped the ILK chromosomal locus to region 11p15, it was determined that a subset of breast carcinomas displays LOH for markers in chromosomal region 11p15.5. This region has also been implicated in an inherited form of cardiac arrythmia, the long QT syndrome. A high level of expression of ILK mRNA indicates an integrin-independent function for ILK in cardiac tissue.

### Example 1 Isolating cDNA of ILK and ILK

A partial cDNA, BIT-9, was isolated in a two-hybrid screen**⁴** using a bait plasmid expressing the cytoplasmic domain of the β₁ integrin subunit. The BIT-9 insert was used to isolate clones from a human placental cDNA library. A 1.8 kb clone, Plac5, was found to contain a high degree of similarity to cDNAs encoding protein kinases (Figure 1 a-c), and recognized a widely expressed transcript of 1.8 kb in Northern blots (Figure 1 d). Deduced amino acid residues 186-451 from Plac5 comprise a domain which is highly homologous with the catalytic domains of a large number of protein tyrosine and serine/threonine kinases (Figure 1b). Residues 33-164 comprise four repeats of a motif originally identified in erythrocyte ankyrin**⁵** (Figure 1 c), likely defining a domain involved in mediating additional protein-protein interactions.**^{6, 7}** Affinity-purified anti-ILK antibodies (see methods described in Example 3) were used in Western blot analyses of mammalian cell extracts, and detected a conserved protein of apparent Mr of 59 kDa (p59^{ILK}, Figure 1 e).

Figure 1 shows yeast two-hybrid cloning, characterization, and expression of ILK. **a,** The full length ILK cDNA, Plac5, was isolated from a human placental library using the BIT-9 insert. Plac5 contains a 1509 bp open reading frame, with a presumptive initiator Met**¹⁷** at nt 157, and an AAUAAA signal 11 bp upstream of the polyadenylation site. *In vitro* transcription and translation of Plac5 in rabbit reticulocyte lysates yielded a protein of apparent Mr of 59 kDa (not shown). **b,** A search**¹⁸** of the PIR protein database indicated homology with protein kinase subdomains I to XI, as identified by Hanks et al.**¹⁹** We note sequence variations in the ILK subdomains I, VIb, and VII, relative to catalytic domains of known protein kinases. Subdomain I (residues 199-213), does not have the typical GXGXXG motif, although this region in ILK is Gly-rich. In subdomain VIb, Asp³²⁸ of ILK may compensate for the lack of the otherwise conserved Asp³¹⁹. In subdomain VII, the DFG triplet is absent in ILK. The integrin binding site maps to amino acid residues 293-451 (BIT-9). The ILK kinase domain is most highly related to the CTR1 kinase of *Arabidopsis thaliana* (30% identity, P < 10⁻¹³). The CTR1, B-raf, Yes and Csk kinase domains are aligned with Plac5. **c,** Amino acid residues 33-164 comprise four contiguous ankyrin repeats, as defined by Lux et. al.**⁵ d,** BIT-9 was used to probe a blot of poly A+ selected RNA (MTN I, Clontech) from various human tissues. **e,** Whole cell lysates of mouse, rat and human cell lines (10 µg/lane) were analyzed by Western blotting with the affinity-purified 92-2 antibody (see description of methods in Example 3). The ILK sequence data are available from GenBank under accession number U40282.

In order to construct integrin 'bait' plasmids**²⁰**, sequences encoding amino acid residues 738-798 of the β₁, and residues 1022-1049 of the α₅ integrin subunits were amplified from full-length cDNAs.**²¹** The primers used were (a) 5' amplification ^{5'} - GGCCGAATTCGCTGGAATTGTTCTTATTGGC -3' and (b) 3' amplification^{5'} - GGCCGGATCCTCATTTTCCCTCATACTTCGG -^{3'}. PCR products were directionally cloned into pEG202, creating the LexA fusion bait plasmids, pEG202β₁INT and pEG202α5INT. pEG202β₁INT and pEG202α₅INT repressed β-gal expression from the pJK101 reporter by 50-60% and 70-75%, respectively, in host strain EGY48 (MATα , his3, trp1, ura3-52, LEU2::pLEU2-LexAop6, constructed by Erica Golemis, Massachussetts General Hospital), confirming nuclear expression of the LexA fusions. Co-transformation of baits with the pSH18-34 reporter verified they were transcriptionally inert (not shown). A galactose-inducible HeLa cDNA interactor library was present on the TRP+ vector, pJG4-5 (constructed by Jeno Gyuris, MGH).
For the β₁ interaction trap, EGY48 was transformed sequentially with pEG202β₁INT, pSH18-34 and pJG4-5, using the lithium acetate protocol**²²** (transformation efficiency = 5-6 x 10⁴/µg). 2x10⁶ primary transformants were screened, of which forty-nine interacting clones were confirmed. The most frequent isolate (31/49) was a 700 bp insert, BIT-9. Retransformation of EGY48 with the BIT-9, pSH18-34, and pEG202β₁INT plasmids resulted in strong β-galactosidase expression, confirming the interaction. An identical screen, using pEG202asINT as bait, resulted in the isolation of 16 positives, none of which were represented in the set of 49 β₁ interactors. Trapped inserts were used to screen WM35 human melanoma λgt10, and human placental λgt11 cDNA libraries, using standard procedures.**²³** cDNA sequencing of multiple clones from each library was done using the dideoxy chain termination method (Sequenase 2.0, U.S. Biochemical). For data analysis we used the Genetics Computer Group software package (version 7.0), and database searches were accomplished via the BLAST**¹⁸** server at the National Center for Biotechnology Information.

### Example 2 Analysis of ILK In Vitro

For analysis of kinase activity in vitro, a bacterially-expressed fusion protein, GST-ILK¹³², was SDS-PAGE band purified, and incubated with [γ-³²P]ATP in the presence or absence of the exogenous substrate myelin basic protein (Figure 2). GST-ILK¹³² autophosphorylated and labelled MBP efficiently in these assays (Figure 2 a). Anti-GST-ILK¹³² (antibody 91-3) immunoprecipitates of PC3 cell lysates were incubated with [γ-³²P]ATP, similar to experiments performed with purified recombinant GST-ILK¹³². ILK immune complexes labelled a protein of apparent Mr of 59kDa (Figure 2 b), corresponding to p59^{ILK}, as well as cellular proteins of apparent Mr 32 kDa and 70 kDa, which may be endogenous ILK substrates (Figure 2 b). We also see cellular phosphoproteins (serine/threonine) of approximately 32 kDa and 70 kDa, in β₁ integrin-specific immune complex kinase assays (not shown).

In ILK immune complex kinase assays a synthetic peptide representing the β₃ cytoplasmic domain was phosphorylated, while a similar peptide representing the β₃ cytoplasmic domain was not detectably labelled by p59^{ILK}. The β₁ peptide selectively inhibited autophosphorylation of ILK in these reactions (Fig. 2b), further indicating a differential interaction of the peptides with ILK. The results demonstrating phosphorylation of synthetic β peptides by endogenous ILK are identical to those seen with recombinant GST-ILK¹³² (not shown), and indicate the potential substrate preference of ILK for the β₁ cytoplasmic tail. This does not, however, necessarily rule out an interaction between ILK and the β₃ integrin cytoplasmic domain. Phosphoamino acid analyses, of labelled p59^{ILK} and MBP from the immune complex kinase assays detected only phosphoserine in both substrates (Fig. 2 c), as was the case for phosphorylation of these substrates by GST-ILK¹³² (not shown). The β₁ peptide was labelled on serine and threonine residues, with approximately equal stoichiometry (Figure 2). As a control, anti-FAK**^{8, 9}** immune complexes from the same lysates were analyzed for phosphorylation of MBP, and phosphotyrosine was readily detected (not shown).

Figure 2 shows i*n vitro* and immune-complex kinase assays. **a,** *In vitro* kinase reactions containing 2 µg of gel-purified GST-ILK¹³², with and without 5 µg of myelin basic protein (MBP, Upstate Biotechnologies, Inc.), were analyzed by 10% SDS-PAGE. **b,** Immune complexes were generated from PC3 whole cell lysates, using affinity-purified 91-3 antibody. Complexes were assayed for kinase activity, with and without addition of 5 µg/reaction of synthetic peptides, representing β₁ or β₃ integrin cytoplasmic domains, **²⁴** or MBP (not shown). Products were analyzed by 15% SDS-PAGE (kDa markers at left), and migration of peptides confirmed by Coomassie Blue staining. **c,** ³²P-labelled products from the anti-ILK immune complex kinase reactions shown in b, were isolated and analyzed for phosphoamino acid content. Anti-FAK**^{8, 9}** immune complex kinase assays demonstrated phosphotyrosine on MBP (not shown).

Protein kinase assays were performed in 50 µl kinase reaction buffer (50 mM HEPES pH 7.0, 10 mM MnCl₂, 10 mM MgCl₂, 2 mM NaF, 1 mM Na₃VO₄). containing 10 µCi [y-³²P]ATP. Reactions were incubated at 30°C for 20 min, and stopped by the addition of SDS-PAGE sample buffer. For assay of recombinant ILK activity, GST-ILK¹³² was adsorbed from bacterial lysates onto glutathione-agarose beads, or GST-ILK¹³² was band-purified from 10% SDS-PAGE gels. For immune complex kinase assays, affinity-purified 91-3 anti-ILK antibody (Fig. 3, Methods) was used to generate immunoprecipitates from NP-40 lysates (150 mM NaCl, 1% (v/v) NP-40, 0.5% (w/v) sodium deoxycholate, 50 mM HEPES pH 7.5, 1 µg/ml each leupeptin and aprotinin, 50 µg/ml phenyl-methylsulfonyl flouride) of PC3 cells. Kinase reaction products were resolved on 10-15% SDS-PAGE gels, transferred to PVDF, and phosphoamino acid analysis performed according to a published protocol.**²⁵**

### Example 3 Association of ILK and b integrin In Mammalian Cells

Immunofluorescence experiments indicated that ILK and β integrin co-localize in focal plaques (not shown). In order to test further for this association in intact mammalian cells, we performed co-immunoprecipitation assays in lysates of PC3 cells, in which integrin expression has been well-characterized.**¹⁰** PC3 cell lysates were immunoprecipitated with specific anti-integrin antibodies,**¹³** and immune complexes analyzed by Western blotting with the anti-ILK antibody, 92-2. The specificities of the anti-ILK antibodies were tested by immunoprecipitation and Western blotting (Figure 3 a, b). Wₑ detected p59^{ILK} in immune complexes obtained with anti-fibronectin receptor (FNR, α₅/α₃ β integrin), and anti-vitronectin receptor (VNR, αᵥβ₃/β₅ integrin) antibodies, but not in those obtained with non-immune serum (Figure 3 c). Three anti-β₁ monoclonal antibodies also co-precipitated p59^{ILK} from PC₃ lysates, confirming the β integrin specificity of p59^{ILK} interaction (Figure 3 d). The detection of p59^{ILK} in anti-VNR immune complexes suggests that ILK may also interact with the β₃ and/or β₅ integrin subunit(s).

Figure 3 shows that antibodies to GST-ILK¹³² recognize p59^{ILK} in integrin co-immunoprecipitations. **a,** Unfractionated polyclonal anti-ILK sera 91-3 (shown) and 92-2 specifically recognize a ³⁵S-methionine, metabolically-labelled cellular protein, of apparent Mr of 59 kDa. A fluorograph is shown (En³Hance, NEN). **b,** Affinity-purified 92-2 antibody was adsorbed with 165 µg of agarose-coupled GST-ILK¹³², or agarose-GST, which preparations were used in parallel Western blots containing 10 µg/lane of whole cell lysates of PC3 cells, Jurkat T-lymphoblasts, or the 60 kDa GST-ILK¹³² . **c,** Polyclonal anti-integrin antibodies, specific for the fibronectin and vitronectin receptors, were used to precipitate surface-biotinylated integrins from PC3 cells, and immune complexes were then analyzed for the presence of p59^{ILK}, by Western blotting with affinity-purified, biotin-labelled 92-2 antibody. This result is representative of six independent experiments. **d,** Anti-β₁ monoclonal antibodies were used in co-precipitation analyses of NP-40 lysates of PC3: lane 1, A_{II}B₂; lane 2, anti-CD29; lane 3, 3S3. Western blotting of anti-β₁ immune complexes with affinity-purified, biotinylated 92-2 antibody (left). This blot was stripped and reprobed with the same concentration of biotinylated 92-2, adsorbed against an excess of GST-ILK¹³² beads (right). We observe co-precipitation of p59^{ILK} using a panel of 11 anti-b₁ monoclonals, but not with an anti-CD44 monoclonal antibody (not shown). The migration of p59^{ILK} was confirmed in parallel lanes containing PC3 whole cell NP-40 lysates. Markers at left, in kDa.

Amino acid residues 132-451 of ILK were expressed as a GST fusion protein, in E. coli. Recombinant GST-ILK¹³² protein was purified and used to inject two rabbits. The resulting antisera, 91-3 and 92-2 (raised by Research Genetics, Inc.), were affinity-purified over a column of CNBr-Sepharose coupled GST-ILK¹³². PC3 cells were metabolically labelled with 100 µCi/ml [³⁵S]methionine/ [³⁵S]cysteine ([³⁵S] ProMix, 1000 Ci/mmol, Amersham), for 18 hours in cysteine/methionine-free MEM. For co-immunoprecipitation experiments PC3 cells were surface-labelled with sulfo-NHS-biotin**²⁶** (Pierce Chemicals), prior to lysis in NP-40 buffer. Polyclonal anti-fibronectin receptor (anti-FNR, Telios A108), and anti-vitronectin receptor (anti-VNR, Telios A109) antibodies were purchased from Gibco/BRL. 1-2 mg of NP-40 lysate was incubated at 4°C, with 2-3 µl/ml anti-FNR or anti-VNR antiserum, or 2 µg/ml of the anti-β₁ monoclonal antibodies A_{II}B₂ (C. Damsky, UC, San Francisco), anti-CD29 (Upstate Biotechnology, Inc.), and 3S3 (J. Wilkins, U Manitoba). Lysates were pre-cleared and immune complexes collected with, Protein A-Sepharose. For Western blotting, RIPA**²⁷** lysates or immune complexes were subjected to 7.5% or 10% SDS-PAGE, and proteins then electrophoretically transferred to polyvinylidene fluoride membranes (Immobilon-P, Millipore). Membranes were blocked in 5% non-fat milk/Tris-buffered saline Tween-20, and incubated with 0.5 µg/ml affinity purified antibodies. Horseradish peroxidase-coupled goat anti-rabbit IgG was used in secondary incubations, followed by detection of reactive bands by enhanced chemiluminescence (ECL, Amersham). For blotting without use of secondary antibody (Fig. 3), affinity-purified 92-2 antibody was labelled with Biotin Hydrazide (Immunopure, Pierce Chemicals), according to the manufacturer's protocol, with visualization by peroxidase-conjugated streptavidin (Jackson ImmunoResearch Laboratories) and ECL. For re-probing, membranes were stripped according to manufacturer's instructions.

### Example 4 Overexpression of ILK Provides Growth Advantage

We next tested for fibronectin-dependent regulation of ILK kinase activity. Plating of rat intestinal epithelial cells, IEC-18,**¹¹** on fibronectin reduced ILK phosphorylation of MBP in immune complex kinase assays, relative to cells plated on plastic, or kept in suspension (Figure 4 a). This fibronectin-dependent reduction of ILK activity was abrogated in IEC-18 cells expressing an activated H-*ras* allele,**^{.11}** indicating that ras transformation disrupts ECM regulation of ILK activity in these cells. An expression vector containing the full-length ILK cDNA, pCMV-ILK, was stably transfected into IEC-18 cells. Twelve stable clones each, of pCMV-ILK and vector control transfectants, were selected and characterized for p59^{ILK} expression levels. Two representative overexpressing subclones, ILK13-Ala3 and -A4a are illustrated (Figure 4b). Overexpression of p59^{ILK} disrupted the epithelial morphology of IEC-18 cells. ILK13 clones were more refractile, and grew on LN, FN and VN with a stellate morphology, in marked contrast to the typical, 'cobble-stone' morphology of the parental and ILK14 cells (Figure 4 c). We plated the ILK13-Ala3 and -A4a subclones, the control transfectants, ILK14-A2C3 and -A2C6, and IEC-18 cells, on varying concentrations of the integrin substrates, laminin (LN), fibronectin (FN) and vitronectin (VN). Adhesion of the ILK14 and IEC-18 cells was equivalent, whereas that of the overexpressing subclones was significantly reduced, on all these substrates (Figure 4 d). Immunoprecipitation analysis indicated that cell surface integrin expression was unaffected (not shown). The effect of p59^{ILK} overexpression on anchorage-independent growth was examined by assaying the colony forming ability of ILK transfectants in soft agarose. In marked contrast to IEC-18 and transfectant controls, four independent p59^{ILK} overexpressing subclones, ILK13- A4a, A1a3, A4d3 and A4Cl2, formed colonies in these assays (Figure 4 e). The proliferative rates of all of these clones on tissue culture plastic were equivalent to control rates.

Figure 4 shows the modulation of ILK,kinase activity by ECM components. **a,** ILK phosphorylation of MBP was assayed in ILK immune complexes, from lysates of IEC-18 intestinal epithelial cells which were harvested from tissue culture plastic and either kept in suspension, or replated on fibronectin, for 1 hour. A H-*ras*-traneformed variant of IEC-18,**¹¹** Ras37 (transfected with Ras^{val12} in pRC/CMV vector), was assayed in parallel. The band shown is MOP. **b,** Expression levels of p59^{ILK} in two representative clones of IEC-18 cells, transfected with an ILK expression construct (ILK13), two vector control clones (ILK14), and the parental IEC-18 cells are presented. The indicated amounts (µg/lane) of whole cell RIPA lysates were run out on 10% SDS-PAGE gels, and p59^{ILK} expression analyzed by Western blotting with affinity-purified 92-2 antibody, **c,** Representative p59^{ILK} overexpressing clone ILK13-A4a, vector control clone ILK14-A2C3, and parental IEC-18 cells were plated on the ECM substrates LN, FN and VN for 1 hour, then fixed, stained with toluidine blue and photographed (40x mag). d, Adhesion of the ILK overexpressing clones to LN, FN and VN was quantified. Key: IEC-18 (black), ILK14-A2C6 (white), ILK13-Ala3 (dark grey), ILK13-A4a (light grey). Results are presented for 10 µg/ml substrate, and are expressed as % adhesion (+/- s. d.) relative to IEC-18, for each substrate. The serial concentrations of ECM showed similar reductions in adhesion of the ILK13 subclones, and ILK14-A2C3 adhesion was identical to that of ILK14-A2C6, on all three substrates. Immunoprecipitation of surface-biotinylated IEC-18, ILK13, and ILK14 subclones, with the anti-FNR and anti-VNR sera, confirmed there was no change in expression of α₅/α₃β₁ and αᵥβ₃/β₅ integrin subunits in the p59^{ILK} overexpressors (data not shown). Data are representative of two independent experiments. **e,** Four ILK13, p59^{ILK} overexpressing clones were plated in soft agarose, and assayed for colony growth after three (experiment 1) and two (experiment 2) weeks. Parent and vector control transfectants were also assayed, and the ras^{val12} transformed clone, Ras-37, was used as a positive control. Bars represent the mean of duplicate determinations. Maximum colonies in IEC-18 and ILK14 cells was 1/field.

The rat intestinal epithelial cell line IEC-18, and a variant of this line transfected with an activated H-*ras*^{val12} allele, expressed from pRC/CMV, were grown on tissue culture plastic in 5% serum-containing medium, washed three times in minimum essential medium (MEM), and harvested with 5 mM EDTA. These were resuspended in 2.5 mg/ml BSA in MEM, and either kept in suspension, or plated on 10 µg/ml fibronectin-coated plates, for 1 hour at 37°C. NP-40 lysates (300 µg) of these cells were immunoprecipitated with affinity-purified 91-3, and immune complex kinase assays (MBP substrate) performed, as described above. IEC-18 were transfected with the expression vector pRC/CMV, containing Plac5 in the forward orientation relative to the CMV promotor. Stable clones were selected in G418, and subcloned through two rounds of limiting dilution. In all, twelve each of ILK and vector control transfectant subclones were isolated. Protein concentrations were determined using the Bradford reagent (Bio-Rad). Two p59^{ILK} overexpressors, ILK13-A1a3 and ILK13-A4a, and two vector transfectant controls, ILK14-A2C3 and -A2C6, were analyzed for effects of ILK overexpression on cell adhesion to ECM substrates. Adhesion was quantified according to published methods.**²⁸**
For colony formation assays 3 x 10⁵ cells were plated in 35mm wells, in 0.3% agarose, as described previously.**²⁹** Ras-37 were plated at 2 x 10³/well. Colonies were counted and scored per field (d = 1 cm) in duplicate wells, and defined as a minimum aggregate of 50 cells.

These results demonstrate that p59^{ILK} overexpression in the IEC epithelial cells provides a growth advantage, in the absence of proliferative signals normally provided by adhesion.

The transduction of extracellular matrix signals through integrins influences intracellular ('outside-in') and extracellular ('inside-out') functions, both of which appear to require interaction of integrin cytoplasmic domains with cellular proteins.^{1**2, 13**} The association of ILK with β₁ integrin subunits, and specific regulation of its kinase activity by adhesion to fibronectin, suggests that p59^{ILK} is a mediator of integrin signalling. Thus the ankyrin repeat motif likely represents a protein interaction module specifying interactions of ILK with downstream, cytoplasmic or cytoskeletal proteins. Reduced ECM adhesion by the p59^{ILK} overexpressing cells is consistent with our observation of adhesion-dependent inhibition of ILK activity, and suggests that p59^{ILK} plays a role in inside-out integrin signalling.
Furthermore the p59^{ILK}-induced, anchorage-independent growth of epithelial cells indicates a role for ILK in mediating intracellular signal transduction by integrins.**¹⁴⁻¹⁶**

### Example 5 The Effect of Anti-ILK On Cell Migration

The role of ILK in cell motility has important implications for normal physiological processes such as inflammation and wound healing, as well as pathological conditions involving tumour invasiveness and metastatic tumour spread, or osteoporosis (bone is essentially an extracellular matrix secreted by osteoblast, or bone-forming cells, and this deposition can be modulated by integrin expression levels and function). Cell motility is a dynamic process, which is dependent on integrin-ECM interactions. The "on-off" switch function of protein kinases provides an ideal mechanism for the dynamic regulation of integrin affinity states for ECM substrates. Thus we are currently assaying the effect on cell migration of microinjecting highly specific anti-ILK antibodies (thereby inhibiting ILK function) into the cell's cytoplasm. Initially these effects will be assayed in endothelial cells plated on solid substrata, but will be extended to include studies on cell migration through three-dimensional gels composed of ECM proteins.

### Example 6 Anti-Sense Oligonucleotides to Inhibit ILK Activity

The sequence of ILK cDNA provides information for the design and generation of synthetic oligonucleotides for "anti-sense" inhibition of ILK activity. This term derives from the strategy of employing a reverse complement of the coding, or sense strand of a specific messenger RNA, known as an anti-sense oligonucleotide (AO). By binding to its complementary mRNA, the AO inhibits translation of that mRNA into protein, thereby preventing normal protein accumulation in the cell. It is not possible to predict which region of an mRNA will provide the most efficient translational inhibition, although we will test ILK AO derived from the ILK mRNA sequence closest to the presumptive translational start site, as defined in Fig.1, as this provides the most successful reagents for this.

Regardless of the actual chemistry used to construct the AO, or modifications to an anti-ILK AO to improve its efficiency, the cDNA sequence of ILK provides the information for derivation of a specific AO. The cDNA sequence of ILK is used to design oligonucleotide reagents, known as degenerate primers (due to the degeneracy of the genetic code), for use in polymerase chain reaction (PCR)-based screens for cDNAs structurally related to ILK. Similarly, the ILK cDNA is used to screen for related genes in a more conventional screen of genomic or cDNA libraries, by employing less stringent (i.e. milder) hybridization conditions during screening. In this way, distinct cDNA or DNA sequences significantly related to ILK (> 50% nucleotide identity) can be isolated, and a family of ILK-related kinases identified in a non-random fashion.

### Example 7 Mapping of ILK Chromosomal Locus to Assess Imprinted Copies of Gene

We conduct higher resolution mapping of the ILK chromosomal locus through fluorescent in situ hybridization (FISH) to metaphase (i.e. separated and identifiable) human chromosomes has placed the ILK gene on chromosome 11p15. FISH is known to those skilled in the art. Finer resolution uses known marker genes in this region. The 11p15 region is indicates that certain genes (e.g. insulin-like growth factor 2, IGF2) have been shown to be imprinted (i.e. preferentially expressed from either the maternally or paternally-derived chromosomes). This imprinting effectively provides a functional deletion or "knock-out" of one of the two inherited copies of a gene. Thus mutation of the non-imprinted allele (copy) has a more profound outcome, since no compensatory activity is available from the imprinted allele. Also, 11p15 has been identified as a region subject to loss-of-heterozygosity, or LOH, in a subset of breast tumour patients. LOH results in the loss of one allele, for example by gene deletion, and is a mechanism underlying the contribution of a number of tumour suppressor genes to the development of various cancers (e.g. BRCA1 in breast, DCC in colon carcinoma, and RB1 in retinoblastoma).

Thus ILK cDNA sequence is used to develop DNA reagents for the diagnosis and prognostic indications of a significant subset of breast cancers, and these reagents contribute to the molecular classification of such tumours. As mentioned above, the gene(s) on 11p15 contributing to some inherited cases of long QT syndrome are identified, and the candidacy of ILK,as a causative gene for this cardiac condition are evaluated by looking for alterations in ILK gene structure, in families where 11p15 associations have been made.

### Example 8 Induction of in vivo Tumorigenesis by Overexpression of ILK

Overexpression of ILK down-regulates E-cadherin which is an important epithelial cell adhesion molecule mediating cell-cell interactions (Dedhar et al., unpublished observations). The loss of E-cadherin induced by overexpression of ILK in epithelial cells suggests that ILK may promote tumorigenicity in vivo. To test this, we injected cells expressing varying levels of ILK into athymic nude mice subcutaneously. Mice were inoculated subcutaneously with the cells expressing high (ILK13-Ala3 and A4a) or low (IEC-18 and ILK14-A2C3) levels of ILK (10⁷ cells/mouse in PBS). The mice were monitored for tumor formation at the site of inoculation after three weeks. Tumors arose within three weeks in 50% to 100% of the mice injected with the ILK13 cells (10⁷ cells/mouse) that overexpress ILK, whereas no tumors were detected in the mice that were injected with the same number of the IEC-18 or ILK14 cells expressing lower levels of ILK (Table I). Thus, overexpression of ILK in these epithelial cells promotes tumor formation in vivo.

**TABLE I: Tumorigenicity of ILK Overexpressing IEC-18 Cells**

| Cell Line | Number of Mice with Tumors at 3 weeks |
|---|---|
| IEC-18 | 0/6 |
| ILK14-A2C3 | 0/6 |
| ILK13-Ala3 | 6/6 |
| ILK13-A4a | 3/6 |

### Example 9 Increased expression of ILK in human breast carcinoma

The expression of Integrin Linked Kinase in human breast carcinomas was determined by immunohistochemical straining of paraffin embedded sections from human breast cancer biopsies.
Affinity purified anti-ILK polyclonal antibody was used followed by conjugated secondary antibody. The positive staining observed was completely abolished by absorption of the antibody to ILK-coupled sepharose beads. The photomicrographs represent sections from two tumor samples. A total of 30 samples have been examined so far. In every case ILK expression levels are markedly elevated in tumor tissue compared to normal ducts and lobules. Figure 5A shows a normal region showing well formed ducts with a single layer of epithelial cells. ILK staining is most prominent in epithelial cells. The stroma appears negative. Figure 5B shows ductal carcinoma in situ (DCIS). Multiple cell layers are present with markedly elevated ILK staining in the tumor cells. Invasive carcinoma is depicted in figures 5C and 5D. There is markedly elevated expression of ILK compared to the normal tissue shown in figure 5A.

The present invention has been described in terms of particular embodiments found or proposed by the present inventor to comprise preferred modes for the practice of the invention. It will be appreciated by those of skill in the art that, in light of the present disclosure, numerous modifications and changes can be made in the particular embodiments exemplified without departing from the intended scope of the invention. For example, due to codon redundancy, changes can be made in the underlying DNA sequence without affecting the protein sequence. Moreover, due to biological functional equivalency considerations, changes can be made in protein structure without affecting the biological action in kind or amount. All such modifications are intended to be included within the scope of the appended claims.

### REFERENCES

**1.** Damsky C.H., and Werb Z. Curr. Opin. Cell Biol. 4, 772-781 (1992).
**2.** Hynes R.O. Cell 69, 11-25 (1992).
**3.** Clark E.A. and Brugge J.S. Science 268, 233-239 (1995).
4. Fields S. and Song O. Nature 340, 245-246 (1989).
**5.** Lux S.E., John K.M. and Bennett V. Nature 344, 36-42 (1990).
**6.** Inoue J.-I., et al. Proc. Natl. Acad. Sci. U.S.A. 89, 4333-4337 (1992).
**7.** Lukas J., et al. Nature 375, 503-506 (1993).
**8.** Schaller M.D., et al. Proc. Natl. Acad. Sci. U.S.A. 89, 5192-5196 (1992).
**9.** Hanks, S.K., Calalb M.B., Harper M.C. and Patel S.K. Proc. Natl. Acad. Sci. U.S.A. 89, 8481-8491 (1992).
**10.** Dedhar S., Saulnier R., Nagle R. and Overall C.M. Clin. Exp. Metastasis 11, 391-400 (1993).
**11.** Filmus J., et al., Oncogene 9, 3627-3633 (1994).
**12.** O'Toole T.E., et al. J. Cell Biol. 124, 1047-1059 (1994).
**13.** Chen Y.-P.,et al., J. Biol. Chem. 269, 18307-18310 (1994).
**14.** Kapron-Bras C., Fitz-Gibbon L., Jeevaratnam P., Wilkins J. and Dedhar S. J. Biol. Chem. 268, 20701-20704 (1993).
**15.** Chen Q., Kinch M.S., Lin T.H., Burridge K. and Juliano R.L. J. Biol. Chem. 269, 26602-26605 (1994).
**16.** Schlaepfer D.D., Hanks S.K., Hunter T. and van der Geer P. Nature 372, 786-791 (1994).
**17.** Kozak M. Cell 44, 283-292 (1986).
**18.** Altschul S.F., Gish W., Miller W., Myers E.W., and Lipman D.J. (1990) Basic alignment search tool. J. Mol. Biol. 215, 403-410.
**19.** Hanks S.K., Quinn A.M. and Hunter T. Science 241, 42-52 (1988).
**20.** Zervos A.S., Gyuris J. and Brent R. Cell 72, 223-232 (1993).
**21.** Argraves W.S.,et al. J. Cell Biol. 105, 1183-1190 (1987).
**22.** Gietz D., St. Jean A., Woods R.A. and Schiestl R.H. Nucl. Acids Res. 20, 1425 (1992).
**23.** Sambrook J., Fritsch E.F. and Maniatis T. Molecular Cloning: A laboratory manual, 2nd ed. (Cold Spring Harbor Laboratory Press, New York, 1989).
**24.** Otey C.A., Pavalko F.M. and Burridge K. J. Cell Biol. 111, 721-729 (1990).
**25.** Cooper J.A., Sefton B.M. and Hunter T. Methods Enzymol. 99, 387-402 (1983).
**26.** Stephens L.C., Sonne J.E., Fitzgerald M.L. and Damsky C.H. J. Cell Biol. 123, 1607-1620 (1993).
**27.** Harlow E. and Lane D. Antibodies: A Laboratory Manual. (Cold Spring Harbor Laboratory Press, New York, 1988).
**28.** Leung-Hagesteijn C.Y., Milankov K., Michalak M., Wilkins J. and Dedhar S. J. Cell Sci. 107, 589-600 (1994).
**29.** Buick, R.N., Filmus J. and Quaroni, A. Exp. Cell Res. 170, 300-309 (1987).

## Claims

1. An inhibitor of integrin linked kinase selected from;
(a) an antibody to the ILK molecule encoded by the nucleic acid sequence of Figure 1A which is capable of inhibiting ILK function; and
(b) a synthetic anti-sense DNA oligonucleotide that is complementary to Figure 1A and capable of inhibiting ILK function.

2. The inhibitor of claim 1 which is a synthetic anti-sense DNA oligonucleotide.

3. An *in vitro* assay method for screening a potential inhibitor of an ILK molecule which is dysregulated in a disease in a mammal selected from cancer, including leukaemia and solid tumours; chronic inflammatory disease, including arthritis; osteoporosis and cardiovascular disease, which method comprises:
bringing an ILK molecule into contact with a potential inhibitor; and
determining the degree to which the potential inhibitor is able to inhibit the activity of the ILK molecule;
wherein the ILK molecule is an isolated and purified serine/threonine integrin-linked kinase encoded by, or having the sequence of, the nucleic acid of Figure 1A.

4. A method according to claim 3 wherein said disease is breast cancer.

5. A method according to claim 3 wherein said disease is chronic inflammatory disease or cardiovascular disease.

6. An inhibitor as defined in claim 1 for use in the treatment of cancer or chronic inflammatory disease in a mammal.

7. An inhibitor as defined in claim 2 for use in the treatment of cancer or chronic inflammatory disease in a mammal.

8. A method for the diagnosis of a disease selected from chronic inflammatory disease including arthritis, osteoporosis and cardiovascular disease, said method comprising bringing a nucleic acid of Figure 1A or complementary to Figure 1A into contact with a sample and determining amplified ILK DNA or increased expression of one or more ILK encoding nucleic acid molecules in said sample.

9. A method according to claim 8 wherein the nucleic acid molecule is selected from mRNA, cDNA, sense DNA, anti-sense DNA, single-stranded DNA and double stranded DNA.

10. A method for the diagnosis of a disease selected from chronic Inflammatory disease including arthritis, osteoporosis and cardiovascular disease, said method comprising bringing an antibody to the ILK molecule encoded by the nucleic acid sequence of Figure 1A into contact with a sample and determining increased expression of ILK protein in said sample.

11. A method according to any one of claims 8 to 10 for the diagnosis of chronic inflammatory disease or cardiovascular disease.

## Patentansprüche

1. Inhibitor von Integrin-gebundener Kinase, ausgewählt aus:
(a) einem Antikörper gegen das durch die Nucleinsäuresequenz aus Fig. 1A kodierte ILK-Molekül, welcher zur Hemmung von ILK-Funktion in der Lage ist;
(b) einem synthetischen Antisense-DNA-Oligonucleotid, das komplementär zu Fig. 1A ist und zur Hemmung von ILK-Funktion in der Lage ist.

2. Inhibitor nach Anspruch 1, der ein synthetisches Antisense-DNA-Oligonucleotid ist.

3. In-vitro-Testverfahren zum Screenen eines potenziellen Inhibitors eines ILK-Moleküls, das in einer aus Krebs, einschließlich Leukämie und soliden Tumoren; chronischen Entzündungserkrankungen, einschließlich Arthritis; Osteoporose und kardiovaskulären Erkrankungen ausgewählten Erkrankung bei einem Säugetier fehlgesteuert ist, wobei das Verfahren Folgendes umfasst:
das In-Kontakt-Bringen eines ILK-Moleküls mit einem potenziellen Inhibitor; und
das Bestimmen des Ausmaßes, in dem der potenzielle Inhibitor in der Lage ist, die Aktivität des ILK-Moleküls zu hemmen;
wobei das ILK-Molekül eine isolierte und gereinigte Serin/Threonin-Integrin-gebundene Kinase ist, für welche die Nucleinsäure aus Fig. 1A kodiert oder die deren Sequenz aufweist.

4. Verfahren nach Anspruch 3, wobei die Erkrankung Brustkrebs ist.

5. Verfahren nach Anspruch 3, wobei die Erkrankung eine chronische Entzündungserkrankung oder eine kardiovaskuläre Erkrankung ist.

6. Inhibitor nach Anspruch 1 zur Verwendung bei der Behandlung von Krebs oder einer chronischen Entzündungserkrankung bei einem Säugetier.

7. Inhibitor nach Anspruch 2 zur Verwendung bei der Behandlung von Krebs oder einer chronischen Entzündungserkrankung bei einem Säugetier.

8. Verfahren zur Diagnose einer Erkrankung, die aus chronischen Entzündungserkrankungen, einschließlich Arthritis, Osteoporose und kardiovaskulären Erkrankungen ausgewählt ist, wobei das Verfahren das In-Kontakt-Bringen einer Nucleinsäure aus Fig. 1A oder einer zu Fig. 1A komplementären Nucleinsäure mit einer Probe und das Bestimmen von amplifizierter ILK-DNA oder erhöhter Expression eines oder mehrerer für ILK kodierenden Nucleinsäuremoleküle in der Probe umfasst.

9. Verfahren nach Anspruch 8, wobei das Nucleinsäuremolekül aus mRNA, cDNA, Sense-DNA, Antisense-DNA, einzelsträngiger DNA und doppelsträngiger DNA ausgewählt ist.

10. Verfahren zur Diagnose einer Erkrankung, die aus chronischen Entzündungserkrankungen, einschließlich Arthritis, Osteoporose und kardiovaskulären Erkrankungen ausgewählt ist, wobei das Verfahren das In-Kontakt-Bringen eines Antikörpers gegen das durch die Nucleinsäuresequenz aus Fig. 1A kodierte ILK-Molekül mit einer Probe und das Bestimmen erhöhter ILK-Protein-Expression in dieser Probe umfasst.

11. Verfahren nach einem der Ansprüche 8 bis 10 zur Diagnose einer chronischen Entzündungserkrankung oder kardiovaskulären Erkrankung.

## Revendications

1. Inhibiteur d'une kinase liée à une intégrine, sélectionné parmi:
(a) un anticorps à la molécule ILK codé par la séquence d'acides nucléiques de la Figure 1A qui est apte à inhiber la fonction ILK; et
(b) un oligonucléotide d'ADN anti-sens synthétique qui est complémentaire à la Figure 1A et qui est apte à inhiber la fonction ILK.

2. Inhibiteur selon la revendication 1, qui est un oligonucléotide d'ADN anti-sens synthétique.

3. Méthode d'essai *in vitro* pour cribler un inhibiteur potentiel d'une molécule ILK qui est déréglée dans une maladie chez un mammifère sélectionné parmi le cancer, incluant la leucémie et les tumeurs solides; maladies inflammatoires chroniques, incluant l'arthrite; ostéoporose et maladie cardiovasculaire, ladite méthode comprend:
amener une molécule ILK en contact avec un inhibiteur potentiel; et
déterminer le degré selon lequel l'inhibiteur potentiel est apte à inhiber l'activité de la molécule ILK;
où la molécule ILK est une kinase liée à l'intégrine sérine/thréonine purifiée codée par, ou ayant la séquence de l'acide nucléique de la Figure 1A.

4. Méthode selon la revendication 3, dans laquelle ladite maladie est le cancer du sein.

5. Méthode selon la revendication 3, dans laquelle ladite maladie est une maladie inflammatoire chronique ou une maladie cardiovasculaire.

6. Inhibiteur selon la revendication 1, pour utilisation dans le traitement du cancer ou d'une maladie inflammatoire chronique dans un mammifère.

7. Inhibiteur selon la revendication 2, pour une utilisation dans le traitement du cancer ou d'une maladie inflammatoire chronique chez un mammifère.

8. Méthode de diagnostic d'une maladie sélectionnée parmi la maladie inflammatoire chronique incluant l'arthrite, l'ostéoporose et la maladie cardiovasculaire, ladite méthode comprenant l'amenée d'un acide nucléique de la Figure 1A ou complémentaire à la Figure 1A en contact avec un échantillon et la détermination de l'ADN ILK amplifiée ou l'expression accrue d'une ou de plusieurs ILK codant pour les molécules d'acide nucléique dans ledit échantillon.

9. Méthode selon la revendication 8, dans laquelle la molécule d'acide nucléique est sélectionnée parmi ARNm, ADNc, ADN sens, ADN anti-sens, ADN à brin simple et ADN à brin double.

10. Méthode pour la diagnostic d'une maladie sélectionnée parmi la maladie inflammatoire chronique incluant l'arthrite, l'ostéoporose et la maladie cardiovasculaire, ladite méthode comprenant l'amenée d'un anticorps à la molécule d'ILK codée par la séquence d'acides nucléiques de la Figure 1A en contact avec un échantillon et la détermination de l'expression accrue de la protéine d'ILK dans ledit échantillon.

11. Méthode selon l'une quelconque des revendications 8 à 10, pour le diagnostic d'une maladie inflammatoire chronique ou d'une maladie cardiovasculaire.
